# EUROPÄISCHE PATENTSCHRIFT

(11) **EP 2 142 169 B1**
(45) Veröffentlichungstag und Bekanntmachung des Hinweises auf die Patenterteilung: **26.10.2011**
(21) Anmeldenummer: 08749090.0
(22) Anmeldetag: 24.04.2008
(51) Int. Cl.: A61K 9/00, A61K 31/00, A61K 47/10, A61K 47/34

(54) **WÄSSRIGE PHARMAZEUTISCHE ZUBEREITUNG**
AQUEOUS PHARMACEUTICAL PREPARATION
PRÉPARATION PHARMACEUTIQUE AQUEUSE

(30) Priorität: 10.05.2007 DE 102007021862
(43) Veröffentlichungstag der Anmeldung: 13.01.2010
(73) Patentinhaber: Merck Patent GmbH, 64293 Darmstadt (DE)
(72) Erfinder: BAUER, Holger, 69126 Heidelberg (DE); RASENACK, Norbert, 79576 Weil am Rhein (DE); AMMER, Kerstin, 64283 Darmstadt (DE); KEMMERER, Bianka, 63500 Seligenstadt (DE); HEIL, Kirstin, 64291 Darmstadt (DE)
(86) Internationale Anmeldenummer: PCT/EP2008/003291
(87) Internationale Veröffentlichungsnummer: WO 2008/138459

(56) Entgegenhaltungen:
- EP-A- 1 666 069
- WO-A-2005/063735
- WO-A-2007/147480

## Beschreibung

Die vorliegende Erfindung betrifft eine wässrige pharmazeutische Zubereitung enthaltend 1-(2-Dimethylamino-ethyl)-3-((2R,4aS,5R,10bS)-5-phenyl-9-trifluoromethyl-3,4,4a,5,6,10b-hexahydro-2H-pyrano[3,2-c]chinolin-2-ylmethyl)-harnstoff als Wirkstoff, eine flüssige pharmazeutische Zubereitung, die für die Herstellung der wässrigen pharmazeutischen Zubereitung geeignet ist sowie Verfahren zur Herstellung der Zubereitungen.

1-(2-Dimethylamino-ethyl)-3-((2R,4aS,5R,10bS)-5-phenyl-9-trifluoromethyl-3,4,4a,5,6,10b-hexahydro-2H-pyrano[3,2-c]chinolin-2-ylmethyl)-harnstoff ist ein spezifischer Inhibitor des mitotischen Motor-Proteins Eg5 der folgenden Strukturformel

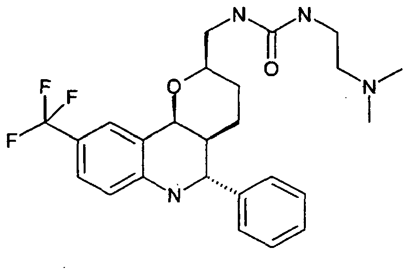

Hemmung des Motor-Proteins Eg5 führt zum Kollaps der Spindelfasern mit der Folge, dass die Chromosomen während der Zellteilung nicht mehr korrekt auf die Tochterzellen aufgeteilt werden. Dies führt insgesamt zu mitotischem Arrest und damit zum Absterben der sich teilenden Zellen. Auf Grund der spezifischen Funktion von Eg5 bei der Zellteilung (Mitose) betrifft die Wirkung hauptsächlich sich schnell teilende und nicht vollständig ausdifferenzierte Zellen.

1-(2-Dimethylamino-ethyl)-3-((2R,4aS,5R,10bS)-5-phenyl-9-trifluoromethyl-3,4,4a,5,6,10b-hexahydro-2H-pyrano[3,2-c]chinolin-2-ylmethyl)-harnstoff ist ein viel versprechender Wirkstoff zur Behandlung verschiedener Erkrankungen und Leiden, die mit einer erhöhten Angiogenese einhergehen, wie Krebs, Tumorentstehung, -wachstum und -verbreitung, Arteriosklerose, Augenerkrankungen, choroidale Neovaskularisierung und diabetische Retinopathie, Entzündungserkrankungen, Arthritis, Neurodegeneration, Restenose, Wundheilung oder Transplantatabstoßung. Von besonderem Interesse ist die Verwendung dieses Wirkstoffes zur Therapie oder Prophylaxe von Krebserkrankungen.

Vor- und nachstehend bedeutet "Wirkstoff" 1-(2-Dimethylamino-ethyl)-3-((2R,4aS,5R,10bS)-5-phenyl-9-trifluoromethyl-3,4,4a,5,6,10b-hexahydro-2H-pyrano[3,2-c]chinolin-2-ylmethyl)-harnstoff.

Soweit der Wirkstoff in der Intensivmedizin eingesetzt wird, wie z. B. bei der Behandlung von Krebserkrankungen, ist es bevorzugt diesen parenteral als Lösung zu verabreichen. WO 2005/063735 beschreibt, dass der Wirkstoff sowie die Salze, Solvate und physiologisch funktionellen Derivate davon, parenteral verabreicht werden können. Zu den an die parenterale Verabreichung angepassten pharmazeutischen Formulierungen gehören wässrige und nichtwässrige sterile Injektionslösungen, die Antioxidantien, Puffer, Bakteriostatika und Solute enthalten. Für die therapeutische Anwendung sind daher parenteral verabreichbare, insbesondere wässrige Lösungen des Wirkstoffes wünschenswert. Geeignete wässrige Lösungen des Wirkstoffes sollten den jeweiligen Therapieerfordernissen angepasst sein, insbesondere sollten sie den Wirkstoff in der therapeutisch erforderlichen Menge enthalten und eine hinreichende Lagerstabilität aufweisen.

Versuche zur Herstellung parenteral verabreichbarer wässriger Lösungen des Wirkstoffs haben jedoch gezeigt, dass die Verbindung ausgesprochen instabil ist. So wird der Wirkstoff in wässriger Lösung, beispielsweise einer Wirkstofflösung in physiologischer Kochsalzlösung, bereits nach wenigen Stunden Lagerung bei Raumtemperatur so stark abgebaut (ca. 20% Degradation nach 2 Stunden), dass er für die therapeutische Verabreichung am Menschen nicht mehr geeignet ist.

Auf Grund des schnellen Abbaus kann die reproduzierbare Verabreichung des Wirkstoffs als wässrige Lösung in der jeweils erforderlichen Menge und Qualität in der Praxis nicht sichergestellt werden. Weiterhin geht der in wässriger Lösung erfolgende Wirkstoffabbau mit einer Zunahme an Abbauprodukten einher, die aus toxikologischer Sicht unerwünscht sind.

Auf Grund der hohen Geschwindigkeit des Wirkstoffabbaus können diese Probleme auch nicht dadurch vermieden werden, indem die wässrige Wirkstofflösung zeitnah vor deren Verabreichung hergestellt wird. Dieses Verfahren hätte zudem noch den Nachteil, dass es mit einem deutlich erhöhten logistischen Aufwand verbunden wäre und daher im Klinikalltag kaum umsetzbar ist. Die Instabilität des Wirkstoffs in wässriger Lösung stellt somit die parenterale Verabreichung von wässrigen Lösungen des Wirkstoffs insgesamt in Frage.

Es wurden umfangreiche Versuche durchgeführt, um eine wässrige pharmazeutische Zubereitung zur Verfügung zu stellen, die ausreichend stabil ist. Beispielsweise wurde erfolglos versucht die Lagerstabilität über Einstellung des pH-Wertes oder durch Zusatz von Puffermedien zu verbessern.

Es war Aufgabe der vorliegenden Erfindung eine wässrige, parenteral verabreichbare Zubereitung für 1-(2-Dimethylamino-ethyl)-3-((2R,4aS,5R,10bS)-5-phenyl-9-trifluoromethyl-3,4,4a,5,6,10b-hexahydro-2H-pyrano[3,2-c]chinolin-2-ylmethyl)-harnstoff zur Verfügung zu stellen, die über eine hinreichende Lagerstabilität verfügt. Um parenteral verabreichbar zu sein, sollte die wässrige pharmazeutische Zubereitung zudem keine toxikologisch bedenklichen Hilfsstoffe enthalten.

Überraschenderweise konnte eine diesen Anforderungen entsprechende Zubereitung zur Verfügung gestellt werden, wenn diese neben dem Wirkstoff mindestens ein nichtionisches Tensid und mindestens ein mindestens eine Hydroxyl-Gruppe enthaltendes organisches Lösungsmittel enthält. Gegenstand der vorliegenden Erfindung ist daher eine wässrige pharmazeutische Zubereitung enthaltend 1-(2-Dimethylamino-ethyl)-3-((2R,4aS,5R,10bS)-5-phenyl-9-trifluoromethyl-3,4,4a,5,6,10b-hexahydro-2H-pyrano[3,2-c]chinolin-2-ylmethyl)-harnstoff als Wirkstoff, mindestens ein nichtionisches Tensid und mindestens ein mindestens eine Hydroxyl-Gruppe enthaltendes organisches Lösungsmittel.

Als "wässrig" werden vor- und nachstehend Wasser und Gemische von Wasser mit anderen Lösungsmitteln, insbesondere organischen Lösungsmitteln, verstanden. Sind im Wasser oder in den Gemischen von Wasser mit anderen Lösungsmitteln ein oder mehrere Stoffe gelöst, liegen wässrige Lösungen vor. Enthält die wässrige Lösung einen oder mehrere Wirkstoffe und ist diese zur therapeutischen oder prophylaktischen Verwendung am Menschen oder Tier geeignet, liegt eine wässrige pharmazeutische Zubereitung vor. Enthält die wässrige Lösung/wässrige pharmazeutische Zubereitung organische Lösungsmittel, sind vorzugsweise solche enthalten, die für die parenterale Anwendung geeignet sind wie Dimethylsulfoxid (DMSO), insbesondere aber Alkohole wie z. B. Ethanol, 1,2-Propandiol, Glycerol,Polyethylenglykole und Glykofurol.

Wenn eine flüssige Zubereitung ausschließlich organische Lösungsmittel enthält oder die enthaltenen organische Lösungsmittel natürlicherweise und unverdünnt bereits einen gewissen Wasseranteil enthalten (so enthält beispielsweise reines Ethanol einen Wasseranteil von ca. 4 Prozent), liegt keine wässrige Zubereitung vor. Wird einer solchen Zubereitung jedoch Wasser oder eine wässrige Lösung (beispielsweise physiologische Kochsalzlösung) zugefügt, ergibt sich eine wässrige Zubereitung im Sinne der vorliegenden Anmeldung.

Vor- und nachstehend bedeutet "Wasser" das Lösungsmittel Wasser oder eine Lösung mit Wasser als Lösungsmittel, z. B. eine Lösung von Wasser enthaltend Hilfsstoffe wie Isotonisierungsmittel, wie z. B. Natriumchlorid, Natriumphosphate oder Glucose. Ein Beispiel für einen Lösung mit Wasser als alleinigem Lösungsmittel ist physiologische Kochsalzlösung.

1-(2-Dimethylamino-ethyl)-3-((2R,4aS,5R,10bS)-5-phenyl-9-trifluoromethyl-3,4,4a,5,6,10b-hexahydro-2H-pyrano[3,2-c]chinolin-2-ylmethyl)-harnstoff kann in der pharmazeutischen Zubereitung als freie Base oder in Form eines seiner Säureadditionssalze wie z. B. als Hydrobromid, Hydrochlorid, Dihydrochlorid, Acetat, Aspartat, Benzoat, Citrat, Fumarat, Glutamat,

Maleat, Methansulfonat oder Tartrat enthalten sein. Bevorzugt ist der Wirkstoff als freie Base enthalten.

Unter nichtionischen Tensiden werden oberflächenaktive Substanzen verstanden, die im Gegensatz zu ionischen Tensiden in wässriger Lösung nicht zu ionischen Substanzen dissoziieren. Als oberflächenaktive Substanzen haben nichtionische Tenside den für Tenside charakteristischen Aufbau, d. h. sie enthalten mindestens eine polare (hydrophile) funktionelle Gruppe sowie mindestens einen hydrophoben Molekülteil. Im Gegensatz zu ionischen Tensiden, in denen die hydrophile Gruppe sich erst in wässriger Lösung infolge Dissoziation in Ionen ergibt, enthalten nichtionische Tenside mindestens eine polare nichtionische funktionelle Gruppe, die nicht in Ionen dissoziiert. Bevorzugt enthält die wässrige Zubereitung nichtionische Tenside mit einem HLB-Wert >8. HLB ist die Abkürzung für Hydrophile-lipophile Balance (hydrophilic lipophilic balance nach Griffin). Der HLB-Wert beschreibt die hydrophilen/lipophilen (amphiphilen) Eigenschaften nichtionischer Tenside und ist ein dimensionsloser Zahlenwert zwischen 1 und 20, der sich aus dem Verhältnis der Molekülmassen des lipophilen und hydrophilen Anteils im Molekül errechnet. Ein hydrophiler Anteil von 100% entspricht dabei dem Wert 20.

Die erfindungsgemäße wässrige pharmazeutische Zubereitung enthält ein oder mehrere nichtionische Tenside, die toxikologisch gut verträglich sind, insbesondere solche die auch parenteral applizierbar sind. Dies sind insbesondere nichtionische Tenside, die als hydrophile funktionelle Gruppen Hydroxyl-Gruppen und/oder Etherbrücken enthalten. Beispiele für derartige nichtionische Tenside sind Polyoxyethylen-Sorbitan-Fettsäureester, Polyoxyethylen-Polyoxypropylen-Copolymere (Poloxamere), Polyethylenglykol-Fettsäureester und Polyethylenglykol-Hydroxyfettsäureester. Polyoxyethylen-Sorbitan-Fettsäureester (Polysorbate) sind u.a. unter dem Warenzeichen Tween bekannt. Geeignete Polyethylen-Sorbitan-Fettsäureester sind insbesondere Polyoxyethylen(20)-sorbitanmonolaurat, Polyoxyethylen(20)-sorbitanmonopalmitat und Polyoxyethylen(20)-sorbitanmonostearat. Bevorzugt sind Polyoxyethylen(20)-sorbitanmonolaurat (z. B. Tween 20^{®}) und Polyoxyethylen(20)-sorbitanmonooleat (z. B. Tween 80^{®}), hiervon besonders bevorzugt ist Polyoxyethylen(20)-sorbitanmonooleat. Polyoxyethylen-Polyoxypropylen-Copolymere sind auch unter der INCI-Bezeichnung Poloxamer bekannt. Als Polyoxyethylen-Polyoxypropylen-Copolymer besonders bevorzugt ist Poloxamer 188 (CAS 9003-11-6, z. B. Lutrol F68^{®}). Als Polyethylenglykol-Hydroxyfettsäureester bevorzugt ist Polyethylenglykol(15)hydroxystearat (Polyethylenglykol 660 12-hydroxysterate, Solutol HS15^{®}), welches auch unter der Bezeichnung Macrogol(15)hydroxystearat im Europäischen Arzneibuch monographiert ist sowie Polyethylenglykol-ricinoleat (Polyoxyl 35 castor oil, Cremophor EL^{®}). Die wässrige pharmazeutische Zubereitung kann ein oder mehrere nichtionische Tenside enthalten.

Nach einer zweckmäßigen Ausführungsform der Erfindung ist die erfindungsgemäße wässrige pharmazeutische Zubereitung dadurch gekennzeichnet, dass als nichtionische/s Tensid/e Polyethylen-Sorbitan-Fettsäureester, Polyoxyethylen-Polyoxypropylen-Copolymer/e, Polyethylenglykol-Fettsäureester und/oder Polyethylenglykol-Hydroxyfettsäureester enthalten ist/sind.

Nach einer bevorzugten Ausführungsform der Erfindung ist die erfindungsgemäße wässrige pharmazeutische Zubereitung dadurch gekennzeichnet, dass als nichtionische/s Tensid/e, Polyethylenglykol-ricinoleat (z. B. Cremophor EL^{®}), Polyoxyethylen(20)-sorbitanmonooleat (z.B. Tween 80^{®}), Polyoxyethylen(20)-sorbitanmonolaurat (z.B. Tween 20^{®}), Polyoxyethylen-Polyoxypropylen-Blockcopolymere (z. B. Lutrol F68^{®}) und/oder Polyethylenglykol(15)hydroxystearat (z. B. Solutol HS15^{®}) enthalten ist/sind.

Als mindestens eine Hydroxyl-Gruppe enthaltende organische Lösungsmittel einsetzbar sind insbesondere ein- oder mehrwertige Alkohole und/oder Polyethylenglykole. Nach einer zweckmäßigen Ausführungsform der Erfindung ist die wässrige erfindungsgemäße pharmazeutische Zubereitung daher dadurch gekennzeichnet, dass als mindestens eine Hydroxyl-Gruppe enthaltende/s organische/s Lösungsmittel ein- oder mehrwertige/r Alkohol/e und/oder Polyethylenglykol/e enthalten ist/sind. Bevorzugt enthält die erfindungsgemäße wässrige pharmazeutische Zubereitung als Alkohole Ethanol, Propylenglykol, Glycerin, Glykofurol und/oder Polyethylenglykol/e, besonders bevorzugt ist dabei Ethanol.

Als Alkohole und Polyethylenglykole können alle Alkohole und Polyethylenglykole enthalten sein, die mit Wasser mischbar sind und zusammen mit diesem eine einheitliche (wässrige) Phase bilden und die aus toxikologischer Sicht geeignet sind, vorzugsweise solche die auch parenteral verträglich sind.

Unter ein- und mehrwertige Alkoholen werden Kohlenwasserstoffe verstanden in denen ein- oder mehrere Wasserstoffatome durch eine Hydroxyl-Gruppe (OH-Gruppe) ersetzt ist/sind. Beispiele für ein-, zwei- und dreiwertige Alkohole sind Ethanol, Propylenglycol (1,2-Propandiol) bzw. Glycerin (1,2,3-Propantriol). Bevorzugt enthält die erfindungsgemäße wässrige pharmazeutische Zubereitung als Alkohol Ethanol, Propylenglykol, Glycerin, Glykofurol und/oder Polyethylenglykol, besonders bevorzugt ist dabei Ethanol.

Polyethylenglykole sind Polykondensationsprodukte der allgemeinen Formel HO-(CH₂-CH₂-O-)ₙH (mit n = 3 bis 200). In der erfindungsgemäßen wässrigen Zubereitung einsetzbar sind Polyethylenglykole mit einem mittleren Molmasse von 200 bis 1500, bevorzugt mit einer mittleren Molmasse von 200 bis 600. Besonders bevorzugt ist Polyethylenglykol mit einem mittleren Molekulargewicht von 400.

Nach einer bevorzugten Ausführungsform der Erfindung ist daher die wässrige pharmazeutische Zubereitung dadurch gekennzeichnet, dass als mindestens eine Hydroxyl-Gruppe enthaltende/s organische/s Lösungsmittel, Ethanol, Propylenglykol, Glycerin, Glykofurol und/oder Polyethylenglykol enthalten ist/sind. Nach einer besonders bevorzugten Ausführungsform der Erfindung ist die wässrige pharmazeutische Zubereitung dadurch gekennzeichnet, dass als mindestens eine Hydroxyl-Gruppe enthaltendes organisches Lösungsmittel Ethanol enthalten ist.

Nach einer zweckmäßigen Ausführungsform der Erfindung ist die wässrige pharmazeutische Zubereitung dadurch gekennzeichnet, dass diese 0,1 bis 20 Gew.-% nichtionische/s Tensid/e und 0,05 bis 20 Gew.-% mindestens ein mindestens eine Hydroxyl-Gruppe enthaltendes organisches Lösungsmittel enthält.

Nach einer bevorzugten Ausführungsform der Erfindung ist die wässrige pharmazeutische Zubereitung dadurch gekennzeichnet, dass diese 0,2 bis 5 Gew.-% nichtionische/s Tensid/e und 0,1 bis 5 Gew.-% mindestens ein mindestens eine Hydroxyl-Gruppe enthaltendes organisches Lösungsmittel enthält.

Nach einer besonders bevorzugten Ausführungsform der Erfindung ist die wässrige pharmazeutische Zubereitung dadurch gekennzeichnet, dass diese 0,5 bis 2 Gew.-% nichtionische/s Tensid/e und 0,2 bis 2 Gew.-% mindestens ein mindestens eine Hydroxyl-Gruppe enthaltendes organisches Lösungsmittel enthält.

Besonders bevorzugt enthält die wässrige pharmazeutische Zubereitung als nichtionisches Tensid Polyethylenglykol(15)hydroxystearat (z. B. Solutol HS15^{®}) und als mindestens eine Hydroxyl-Gruppe enthaltendes organisches Lösungsmittel Ethanol. Eine besonders bevorzugte Ausführungsform der erfindungsgemäßen wässrigen pharmazeutische Zubereitung ist daher dadurch gekennzeichnet, dass sie als nichtionisches Tensid Polyethylenglykol(15)hydroxystearat und als ein mindestens eine Hydroxyl-Gruppe enthaltendes organisches Lösungsmittel Ethanol enthält. Soll die wässrige pharmazeutische Zubereitung parenteral, z. B. intravenös, verabreicht werden ist die Zubereitung vorzugsweise isotonisch. Enthält sie mehrwertige Alkohole, wie z. B. Glycerin, oder Polyethylenglykole kann Isotonizität durch Anpassung der hiervon enthaltenen Mengen erreicht werden. Sind mehrwertige Alkohole oder Polyethylenglykole nicht oder nicht in der zur Isotonisierung erforderlichen Menge enthalten, kann ein zusätzliches Isotonisierungsmittel, bevorzugt ein physiologisch verträgliches Salz, wie beispielsweise Natriumchlorid oder Natriumphosphat, oder ein physiologisch verträgliches Polyol, wie beispielsweise Glucose oder Glycerin, in einer zur Isotonisierung erforderlichen Konzentration enthalten sein. Als "isotonisch" wird in der vorliegenden Anmeldung eine wässrige Zubereitung bezeichnet, wenn diese eine Osmolalität von 250 bis 350 mOsmol/kg aufweist. Derartige wässrige Zubereitungen können weitgehend schmerzfrei direkt intravenös oder auch intraarteriell verabreicht werden.

Untersuchungen haben ergaben dass der Wirkstoff in der wässrigen pharmazeutischen Zubereitung nur sehr langsam hydrolisiert. Auch bei Lagerung bei Raumtemperatur ist der Wirkstoff in der erfindungsgemäßen wässrigen pharmazeutischen Zubereitung so stabil, dass diese problemlos über mindestens 24 Stunden ab Herstellung parenteral verabreicht werden kann. Wird die wässrige Lösung kühl, zum Beispiel bei Kühlschranktemperatur (2 - 8°C), gelagert, oder wird sie eingefroren, ist die Haltbarkeit noch einmal deutlich verlängert. Die Lagerung der erfindungsgemäßen wässrigen Zubereitung bei Kühlschranktemperatur ist bevorzugt.

Die erfindungsgemäße wässrige pharmazeutische Zubereitung kann hergestellt werden, indem der Wirkstoff und die genannten weiteren Inhaltsstoffe in den genannten Lösungsmitteln gelöst werden. Die Reihenfolge, in der Wirkstoff, Tensid/e und ggf. weitere Hilfsstoffe in dem wässrigen und/oder organischen Lösungsmittel gelöst werden, ist grundsätzlich beliebig variierbar. Die Herstellung kann auch dadurch erfolgen, dass zunächst unterschiedliche Stoffe in unterschiedlichen Lösungsmitteln gelöst werden und die erhaltenen Lösungen anschließend miteinander vereinigt werden.

Nach einer zweckmäßigen Ausführungsform der Erfindung wird die erfindungsgemäße wässrige pharmazeutische Zubereitung dadurch hergestellt, indem der Wirkstoff in mindestens einem mindestens eine Hydroxyl-Gruppe enthaltenden organischen Lösungsmittel, in mindestens einem nichtionischen Tensid oder in einer Mischung aus mindestens einem mindestens eine Hydroxyl-Gruppe enthaltenden organischen Lösungsmittel und mindestens einem nichtionischen Tensid gelöst wird, wobei die vorgenannten Lösungen ggf. noch weitere Hilfsstoffe enthalten können, und die erhaltene Lösungen anschließend, wenn der Wirkstoff in einer Mischung aus mindestens einem mindestens eine Hydroxyl-Gruppe enthaltenden organischen Lösungsmittel und mindestens einem nichtionischen Tensid gelöst ist, mit Wasser, wenn der Wirkstoff in mindestens einem mindestens eine Hydroxyl-Gruppe enthaltenden organischen Lösungsmittel gelöst ist, mit mindestens einem nichtionischen Tensid und Wasser, und wenn der Wirkstoff in mindestens einem nichtionischen Tensid gelöst ist, mit mindestens einem mindestens eine Hydroxyl-Gruppe enthaltenden organischen Lösungsmittel und Wasser vereinigt und vermischt werden.

Liegt der Wirkstoff zunächst als Lösung in mindestens einem nichtionischen Tensid oder in mindestens einem mindestens eine Hydroxyl-Gruppe enthaltenden organischen Lösungsmittel vor können die jeweils noch fehlenden Bestandteile (ein oder mehrere mindestens eine Hydroxyl-Gruppe enthaltende organischen Lösungsmittel und Wasser bzw. ein oder mehrere nichtionische Tenside und Wasser) in beliebiger Reihenfolge nacheinander oder zusammen als Mischung zugegeben werden.

Aus praktischen Erwägungen ist es vorteilhaft die noch fehlenden Bestandteile gemeinsam als Mischung zuzugeben. Liegt der Wirkstoff also zunächst als Lösung in mindestens einem nichtionischen Tensid vor, ist es bevorzugt die erfindungsgemäße wässrige Zubereitung dadurch bereitzustellen, indem diese mit einer Mischung vereinigt und vermischt wird, die mindestens ein mindestens eine Hydroxyl-Gruppe enthaltendes organisches Lösungsmittel und Wasser enthält. Liegt der Wirkstoff zunächst als Lösung in mindestens einem mindestens eine Hydroxyl-Gruppe enthaltendem organischen Lösungsmittel vor, ist es bevorzugt die erfindungsgemäße wässrige Zubereitung dadurch bereitzustellen, indem diese mit einer Mischung vereinigt und vermischt wird, die mindestens ein nichtionisches Tensid und Wasser enthält.

Es hat sich gezeigt, dass der Wirkstoff in Lösung über lange Zeit stabil ist, wenn er in dem organischen Lösungsmittel oder Lösungsmittelgemisch und/oder in dem nichtionischen Tensid oder Tensidgemisch, das/die in der erfindungsgemäßen wässrigen Zubereitung enthalten ist/sind, gelöst ist, d. h. wenn er als Lösung in mindestens einem mindestens eine Hydroxyl-Gruppe enthaltenden organischen Lösungsmittel und/oder in mindestens einem nichtionischen Tensid enthalten ist. Die Langzeitstabilität des Wirkstoffes in einem oder mehreren mindestens eine Hydroxyl-Gruppe enthaltenden organischen Lösungsmittel/n, in einem oder mehreren nichtionischen Tensid/en oder in einer Mischung aus einem oder mehreren mindestens eine Hydroxyl-Gruppe enthaltenden organischen Lösungsmittel/n und einem oder mehreren nichtionischen Tensid/en, erlaubt es somit den Wirkstoff in diesen Lösungen über lange Zeit stabil zu lagern (Lagerzeit > 1 Jahr). Aus einer solchen lagerstabilen Lösung, d. h. aus einer Lösung des Wirkstoffes in einem oder mehreren mindestens eine Hydroxyl-Gruppe enthaltenden organischen Lösungsmittel/n, in einem oder mehreren nichtionischen Tensid/en oder in einer Mischung aus einem oder mehreren mindestens eine Hydroxyl-Gruppe enthaltenden organischen Lösungsmittel/n und einem oder mehreren nichtionischen Tensid/en, kann dann die erfindungsgemäße wässrige Lösung, wie beschrieben, dadurch erhalten werden, indem diese mit den jeweils noch fehlenden Bestandteilen vereinigt und gemischt werden.

Mischen von Lösungen ist eine einfache Operation, die auch in der Klinik ohne Probleme durchgeführt werden kann. Somit ist es möglich den Wirkstoff als Lösung in einem organischen Lösungsmittel oder Lösungsmittelgemisch und/oder in dem nichtionischen Tensid oder Tensidgemisch über lange Zeiträume stabil zu lagern und die erfindungsgemäße wässrige pharmazeutische Zubereitung je nach Bedarf erst zeitnah vor deren parenteralen Verabreichung herzustellen. Auf Grund der bereits erwähnten Stabilität der derart erhaltenen wässrigen pharmazeutische Zubereitung muss diese aber nicht direkt verabreicht werden sondern kann problemlos bis zu deren Verabreichung zwischengelagert werden, bei Raumtemperatur über mindestens einen Tag, bei Kühlschranktemperatur (2 - 8°C) über mindestens eine Woche. Selbstverständlich ist es auch möglich die erhaltene Lösung direkt nach deren Herstellung zu verabreichen.

Wie oben beschrieben, ist die Zubereitung enthaltend den Wirkstoff als organische Lösung in einem oder mehreren mindestens eine Hydroxyl-Gruppe enthaltenden organischen Lösungsmittel/n, in einem oder mehreren nichtionischen Tensid/en oder in einer Mischung aus einem oder mehreren mindestens eine Hydroxyl-Gruppe enthaltenden Lösungsmittel/n und einem oder mehreren nichtionischen Tensid/en sind über lange Zeit stabil und ermöglicht es die erfindungsgemäße wässrige Zubereitung in einfacher Weise zeitnah vor deren Verabreichung herzustellen. Gegenstand der Erfindung ist daher auch eine flüssige pharmazeutische Zubereitung geeignet zur Herstellung der erfindungsgemäßen wässrigen pharmazeutischen Zubereitung, die dadurch gekennzeichnet ist, dass diese mindestens ein mindestens eine Hydroxyl-Gruppe enthaltendes organisches Lösungsmittel und/oder mindestens ein nichtionisches Tensid enthält. Diese flüssige pharmazeutische Zubereitung kann bis zu 15 Gew.-% Wasser enthalten, bevorzugt sind weniger als 5 Gew.-% Wasser, besonders bevorzugt weniger als 2 Gew.-% Wasser enthalten. Eine möglichst geringer Wasseranteil ist bevorzugt, jedoch kann zumeist, insbesondere auf Grund von in dem eingesetzten Tensid enthaltendem Wasser in der flüssigen Zubereitung, ein geringer Wasseranteil nicht völlig ausgeschlossen werden.

Nach einer bevorzugten Ausführungsform enthält die flüssige pharmazeutische Zubereitung geeignet zur Herstellung der erfindungsgemäßen wässrigen pharmazeutischen Zubereitung, mindestens ein mindestens eine Hydroxyl-Gruppe enthaltendes organisches Lösungsmittel und mindestens ein nichtionisches Tensid.

Nach einer bevorzugten Ausführungsform der Erfindung ist die wasserfreie Lagerform des Wirkstoffs (langzeitstabile Lösung) dadurch gekennzeichnet, dass als mindestens eine Hydroxyl-Gruppe enthaltende/s organische/s Lösungsmittel, Ethanol, Propylenglykol, Glycerin, Glykofurol und/oder Polyethylenglykol sowie als nichtionische/s Tensid/e Polyethylenglykol-ricinoleat (z. B. Cremophor EL^{®}), Polyoxyethylen(20)-sorbitanmonooleat (z. B. Tween 80^{®}), Polyoxyethylen(20)-sorbitanmonolaurat (z. B. Tween 20^{®}), Polyoxyethylen-Polyoxypropylen-Blockcopolymere (z. B. Lutrol F68^{®}) und/oder Polyethylenglykol(15)hydroxystearat (z. B. Solutol HS15^{®}) enthalten ist/sind.

Sind organische/s Lösungsmittel und nichtionische/s Tensid/e enthalten, enthält die flüssige pharmazeutische Zubereitung 5 bis 95 Gew.-% mindestens eine Hydroxyl-Gruppe enthaltende/s organische/s Lösungsmittel und 5 bis 95 Gew.-% nichtionische/s Tensid/e. Gegenstand der Erfindung ist daher eine flüssige pharmazeutische Zubereitung, die dadurch gekennzeichnet ist, dass diese 5 bis 95 Gew.-% mindestens eine Hydroxyl-Gruppe enthaltende/s organische/s Lösungsmittel und 5 bis 95 Gew.-% nichtionische/s Tensid/e enthält.

Bevorzugt enthält die flüssige pharmazeutische Zubereitung 30 bis 70 Gew.-% mindestens eine Hydroxyl-Gruppe enthaltende/s organische/s Lösungsmittel und 70 bis 30 Gew.-% nichtionische/s Tensid/e, besonders bevorzugt 45 bis 55 Gew.-% mindestens eine Hydroxyl-Gruppe enthaltende/s organische/s Lösungsmittel und 45 bis 55 Gew.-% nichtionische/s Tensid/e. Nach einer bevorzugten Ausführungsform der Erfindung ist die flüssige pharmazeutische Zubereitung daher dadurch gekennzeichnet, dass diese 70 bis 30 Gew.-% mindestens eine Hydroxyl-Gruppe enthaltende/s organische/s Lösungsmittel und 30 bis 70 Gew.-% nichtionische/s Tensid/e enthält. Nach einer besonders bevorzugten Ausführungsform ist die flüssige pharmazeutische Zubereitung dadurch gekennzeichnet, dass diese 45 bis 55 Gew.-% mindestens eine Hydroxyl-Gruppe enthaltende/s organische/s Lösungsmittel und 45 bis 55 Gew.-% nichtionische/s Tensid/e enthält.

Nach einer zweckmäßigen Ausführungsform der Erfindung erfolgt die Herstellung der wässrigen pharmazeutischen Zubereitung durch Mischen einer den Wirkstoff, mindestens ein nichtionisches Tensid und mindestens ein mindestens eine Hydroxyl-Gruppe enthaltendes Lösungsmittel enthaltenden flüssigen Zubereitung mit Wasser.

Gegenstand der Erfindung ist auch ein Verfahren zur Herstellung der wässrigen pharmazeutischen Zubereitung, das dadurch gekennzeichnet ist, dass die flüssige pharmazeutische Zubereitung mit der/den jeweils fehlenden Flüssigkeit/en, d. h. mit Wasser, mit Wasser und mindestens einem nichtionischen Tensid oder mit Wasser und mindestens einem mindestens eine Hydroxyl-Gruppe enthaltenden organischem Lösungsmittel vereinigt wird/werden.

Enthält die flüssige pharmazeutische Zubereitung, die den Wirkstoff enthält, als flüssigen Bestandteil nur organische/s Lösungsmittel oder nur nichtionische/s Tensid/e, ist es bevorzugt, die erfindungsgemäße wässrige pharmazeutische Zubereitung dadurch bereitzustellen, indem diese jeweils mit Mischungen der fehlenden flüssigen Bestandteile, d. h. mit einer Mischung aus Wasser und mindestens einem nichtionischen Tensid bzw. mit einer Mischung aus Wasser und mindestens einem mindestens eine Hydroxyl-Gruppe enthaltenden organischen Lösungsmittel vereinigt wird. Gegenstand der Erfindung ist daher auch ein Verfahren zur Herstellung einer wässrigen pharmazeutischen Zubereitung, das dadurch gekennzeichnet ist, dass die flüssige pharmazeutische Zubereitung, wenn sie als flüssigen Bestandteil nur mindestens ein mindestens eine Hydroxyl-Gruppe enthaltendes organisches Lösungsmittel oder nur mindestens ein nichtionisches Tensid enthält, jeweils mit einer Mischung der fehlenden flüssigen Bestandteile, d. h. mit einer Mischung aus Wasser und mindestens einem nichtionischen Tensid bzw. mit einer Mischung aus Wasser und mindestens einem mindestens eine Hydroxyl-Gruppe enthaltenden organischen Lösungsmittel vereinigt wird.

Die erfindungsgemäße wässrige Lösung kann in besonders einfacher Weise erhalten werden, indem eine flüssige pharmazeutische Zubereitung enthaltend mindestens ein mindestens eine Hydroxyl-Gruppe enthaltendes organisches Lösungsmittel und mindestens ein nichtionisches Tensid einer isotonischen wässrigen Lösung, zum Beispiel physiologische Kochsalzlösung, zugegeben wird. Ist die isotonische wässrige Lösung in einem Infusionsbeutel enthalten, kann dies vorteilhaft dadurch erfolgen, dass die flüssige pharmazeutische Zubereitung in eine Spritze aufgezogen anschließend nach Durchstechen des Beutels mittels einer Kanüle direkt in die isotonische wässrige Lösung hineingespritzt wird. Selbstverständlich können das/die organische/n Lösungsmittel und das/die nichtionische/n Tensid/e auch als getrennte Flüssigkeiten, wobei mindestens eine dieser Flüssigkeiten den Wirkstoff enthält, in gleicher Weise der physiologische Kochsalzlösung hinzugefügt werden, ihre Zugabe als Mischung ist aber bevorzugt.

Die flüssige pharmazeutische Zubereitung und die zur Herstellung der erfindungsgemäßen wässrigen pharmazeutischen Zubereitung weiterhin erforderlichen flüssigen Bestandteile können vorteilhaft zu einer Packung (Kit) zusammengefasst werden. Gegenstand der Erfindung ist daher auch ein Kit enthaltend ein Behältnis mit einer flüssigen pharmazeutischen Zubereitung, die mindestens ein mindestens eine Hydroxyl-Gruppe enthaltendes organisches Lösungsmittel und mindestens ein nichtionisches Tensid enthält, und ein Behältnis mit Wasser geeignet zur Herstellung der erfindungsgemäßen wässrigen pharmazeutischen Zubereitung.

Die Ausführungsbeispiele, ohne hierauf beschränkt zu sein, erläutern die Erfindung.

### Beispiel 1

### Stabilität in verschiedenen Lösungsmitteln und Zubereitungen

Die Stabilität des Wirkstoffes in den verschiedenen Lösungsmitteln und den gefundenen erfindungsgemäßen Zubereitungen wird in Haltbarkeitsstudien überprüft. Hierzu werden die hergestellten Zubereitungen bei verschiedenen Temperaturen eingelagert, zu bestimmten Zeiten ausgelagert und mit geeigneten analytischen Methoden untersucht. Als Klimabedingungen werden Kühlschrank-Temperatur (2-8 °C) und 25°C mit einer relativen Luftfeuchtigkeit (r.F.) von 60% gewählt. Mögliche Instabilitäten äußern sich bei dem Wirkstoff vornehmlich in der Bildung von Abbauprodukten.

Die Ergebnisse der Stabilitätsuntersuchungen sind in Tabelle 1 zusammengestellt.

**Tabelle 1**

| | | | **Gehalt an Wirkstoff [%]** | | | | | |
|---|---|---|---|---|---|---|---|---|
| Lösungsmittelzusammensetzung | **Konz. [mg/ml]** | **Temp. [°C]** | **Start** | **2 Std.** | **6 Std.** | **24 Std.** | **1 Woche** | **1 Monat** |
| Isot. NaCl | * | 25 | 98.8 | 83.4 | 57.3 | | | |
| Isot. NaCl pH 5.0 (Citrat-Puffer) | * | 2-8 | 72.7 | | | | | 9.2 |
| 60% Propylenglycol in isot. NaCl | 5 | 25 | 96.6 | | | | 92.2 | 78.7 |
| 5% Ethanol/5% Solutol HS15 in isot. NaCl | 5 | 25 | 98.2 | | | 97.5** | 93.2 | |
| 5% Ethanol/5% Solutol HS15 in isot. NaCl | 5 | 2-8 | 98.2 | | | 98.4** | 98.2 | |
| 0,8% EtOH/0,8% Solutol HS15 in isot. NaCl | 0,6 | 25 | 109,3 | | 108.4 *** | | | |
| 0,5% EtOH/0,5% Solutol HS15 in isot. NaCl | 0,5 | 2-8 | 104.7 | | | 103.3 | 103.1 | |
| 10% Cremophor EL in isot. NaCl | 5 | 25 | 96.7 | | | | | 84.0 |
| 10% Cremophor EL in isot. NaCl | 5 | 2-8 | 96.7 | | | | 96.7 | 93.2 |
| 10% Solutol HS15 in isot. NaCl | 5 | 25 | 98.4 | | | | 95.2 | 85.4 |
| 10% Solutol HS15 in isot. NaCl | 5 | 2-8 | 98.4 | | | | 97.0 | 96.3 |
| 50% EtOH/50% Solutol HS15 | 50 | 25 | 98.3 | | | | 98.2 | 98.4 |
| Ethanol | 50 | 25 | 98.3 | | | | 98.2 | 98.3 |
| 1,2-Propandiol | 50 | 25 | 98.3 | | | | 98.3 | 98.5 |
| Solutol HS15 | 50 | 25 | 97.9 | | | | 97.9 | 98.0 |
| Cremophor EL | 10 | 25 | 97.5 | | | | | 95.2 |

| | | | | | | | | |
|---|---|---|---|---|---|---|---|---|
| * bei Sättigungskonzentration ** 48 Stunden *** 8 Stunden | | | | | | | | |

### Analytische Testmethoden:

Identität, Reinheit und der Gehalt der Zubereitungen enthaltend 1-(2-Dimethylamino-ethyl)-3-((2R,4aS,5R,10bS)-5-phenyl-9-trifluoromethyl-3,4,4a,5,6,10b-hexahydro-2H-pyrano[3,2-c]chinolin-2-ylmethyl)-harnstoff, werden durch Hochleistungsflüssigchromatographie mit UV-Detektion unter Verwendung einer RP-18-Säule im Hochdruckgradientensystem nach Herstellung und im Zuge der Stabilitätsstudien geprüft. Als Extraktionsmedium und mobile Phase werden gepufferte Acetonitril/Wasser-Gemische verwendet.

### Herstellung von flüssigen Zubereitungen, die zur Herstellung der erfindungsgemäßen wässrigen Zubereitung geeignet sind

### Beispiel 2

| | |
|---|---|
| 1-(2-Dimethylaminoethyl)-3-((2R,4aS,5R,10bS)-5-phenyl-9-triflouromethyl-3,4,4a,5,6, 10b-hexahydro-2H-pyrano[3,2-c]chinolin-2-ylmethyl)-harnstoff | 50 mg / ml |
| Ethanol / Polyethylenglykol(15)hydroxystearat | 50/50 (m/m) |

Der Wirkstoff wird vorgelegt, in einem Teil des Ethanols aufgeschlämmt. Anschließend wird das geschmolzene Polyethylenglykol(15)hydroxystearat hinzugegeben und das Ethanol - inkl. Verdunstungseffekte - auf die Zielmenge aufgefüllt. Die Formulierung wird unter aseptischen Bedinungen abgefüllt.

### Beispiel 3

| | |
|---|---|
| 1-(2-Dimethylaminoethyl)-3-((2R,4aS,5R, 10bS)-5-phenyl-9-triflouromethyl-3,4,4a,5,6,10b-hexahydro-2H-pyrano[3,2-c]chinolin-2-ylmethyl)-harnstoff | 10 mg / ml |
| Ethanol / Polyethylenglykol(15)hydroxystearat | 50/50 (m/m) |

Der Wirkstoff wird vorgelegt, in einem Teil des Ethanols aufgeschlämmt. Anschließend wird das geschmolzene Polyethylenglykol(15)hydroxystearat hinzugegeben und das Ethanol - inkl. Verdunstungseffekte - auf die Zielmenge aufgefüllt.

### Beispiel 4

| | |
|---|---|
| 1-(2-Dimethylaminoethyl)-3-((2R,4aS,5R,10bS)-5-phenyl-9-triflouromethyl-3,4,4a,5,6,10b-hexahydro-2H-pyrano[3,2-c]chinolin-2-ylmethyl)-harnstoff | 100 mg / ml |
| Ethanol / Polyethylenglykol(15)hydroxystearat | 50/50 (m/m) |

Der Wirkstoff wird vorgelegt, in einem Teil des Ethanols aufgeschlämmt. Anschließend wird das geschmolzene Polyethylenglykol(15)hydroxystearat hinzugegeben und das Ethanol - inkl. Verdunstungseffekte - auf die Zielmenge aufgefüllt.

### Beispiel 5

| | |
|---|---|
| 1-(2-Dimethylaminoethyl)-3-((2R,4aS,5R,10bS)-5-phenyl-9-triflouromethyl-3,4,4a,5,6,10b-hexahydro-2H-pyrano[3,2-c]chinolin-2-ylmethyl)-harnstoff | 50 mg / ml |
| Ethanol / Polyethylenglykol(15)hydroxystearat / Polyoxyethylen(20)sorbitanmonolaurat | 50/25/25 (m/m) |

Der Wirkstoff wird vorgelegt, in einem Teil des Ethanols aufgeschlämmt. Anschließend wird das geschmolzene Polyethylenglykol(15)hydroxystearat hinzugegeben und das Ethanol - inkl. Verdunstungseffekte - auf die Zielmenge aufgefüllt.

### Beispiel 6

| | |
|---|---|
| 1-(2-Dimethylaminoethyl)-3-((2R,4aS,5R,10bS)-5-phenyl-9-triflouromethyl-3,4,4a,5,6,10b-hexahydro-2H-pyrano[3,2-c]chinolin-2-ylmethyl)-harnstoff | 50 mg / ml |
| Ethanol / Polyoxyethylen(20)sorbitanmonolaurat | 50/50 (m/m) |

Der Wirkstoff wird vorgelegt, in einem Teil des Ethanols aufgeschlämmt. Anschließend wird das Polyoxyethylen(20)sorbitanmonolaurat hinzugegeben und das Ethanol - inkl. Verdunstungseffekte - auf die Zielmenge aufgefüllt.

### Beispiel 7

| | |
|---|---|
| 1-(2-Dimethylaminoethyl)-3-((2R,4aS,5R,10bS)-5-phenyl-9-triflouromethyl-3,4,4a,5,6,10b-hexahydro-2H-pyrano[3,2-c]chinolin-2-ylmethyl)-harnstoff | 50 mg / ml |
| Propylenglycol / Polyoxyethylen(20)sorbitanmonolaurat | 50/50 (m/m) |

Der Wirkstoff wird vorgelegt, in einem Teil des Propylenglycols aufgeschlämmt. Anschließend wird das Polyoxyethylen(20)sorbitanmonolaurat hinzugegeben und das Propylenglycol auf die Zielmenge aufgefüllt.

### Beispiel 8

| | |
|---|---|
| 1-(2-Dimethylaminoethyl)-3-((2R,4aS,5R,10bS)-5-phenyl-9-triflouromethyl-3,4,4a,5,6,1 0b-hexahydro-2H-pyrano[3,2-c]chinolin-2-ylmethyl)-harnstoff | 50 mg / ml |
| Propylenglycol / Polyethylenglykol(15)hydroxystearat | 50/50 (m/m) |

Der Wirkstoff wird vorgelegt, in einem Teil des Propylenglycols aufgeschlämmt. Anschließend wird das geschmolzene Polyethylenglykol(15)hydroxystearat hinzugegeben und das Propylenglycol auf die Zielmenge aufgefüllt.

### Beispiel 9

| | |
|---|---|
| 1-(2-Dimethylaminoethyl)-3-((2R,4aS,5R,10bS)-5-phenyl-9-triflouromethyl-3,4,4a,5,6,10b-hexahydro-2H-pyrano[3,2-c]chinolin-2-ylmethyl)-harnstoff | 50 mg / ml |
| Propylenglycol / Ethanol / Polyethylenglykol(15) Hydroxystearat | 25 / 25 /50 (m/m) |

Der Wirkstoff wird vorgelegt, in einem Teil des Ethanols aufgeschlämmt. Anschließend wird das geschmolzene Polyethylenglykol(15)hydroxystearat hinzugegeben und das Ethanol und Propylenglykol auf die Zielmenge aufgefüllt.

### Herstellung der erfindungsgemäßen wässrigen Zubereitung: Beispiel 10

Grundrezeptur Wirkstoff Produkt-Lösung A:

| | |
|---|---|
| 1-(2-Dimethylaminoethyl)-3-((2R,4aS,5R,10bS)-5-phenyl-9-triflouromethyl-3,4,4a,5,6,10b-hexahydro-2H-pyrano[3,2-c]chinolin-2-ylmethyl)-harnstoff | 50 mg / ml |
| Ethanol / Polyethylenglykol(15)hydroxystearat | 50/50 (m/m) |

### Beispiel 11

Herstellung einer Lösung zur Infusion, enthaltend 20 mg Wirkstoff in 100 ml Infusionslösung:

Zugabe von 0,4 ml oben angegebener Wirkstoff Produkt-Lösung A in einem Infusionsbeutel enthaltend 100 ml 0,9% Kochsalzlösung sowie 3 ml einer Zubereitung Ethanol / Polyethylenglykol(15)hydroxystearat 50/50 (m/m). Diese Zubereitung zeigt Stabilität über die notwenige Zeitspanne (ca. 0.5% Hydrolyse nach 4 Stunden bei 25°C) zwischen Präparation und Infusion. Zum Vergleich: Der reine Wirkstoff in wässriger Lösung bei Raumtemperatur zeigt in vergleichbarer Zeitspanne eine ca. 45%ige Degradation.

### Beispiel 12

Herstellung einer Lösung zur Infusion, enthaltend 100 mg Wirkstoff in 100 ml Infusionslösung:

Zugabe von 2,0 ml oben angegebener Wirkstoff Produkt-Lösung A in einen Infusionsbeutel enthaltend 100 ml 0,9% Kochsalzlösung sowie 1,4 ml einer Zubereitung Ethanol / Polyethylenglykol(15)hydroxystearat 50/50 (m/m).

### Beispiel 13

Herstellung einer Lösung zur Infusion, enthaltend 170 mg Wirkstoff in 100 ml Infusionslösung:

Zugabe von 3,4 ml oben angegebener Wirkstoff Produkt-Lösung A in einen Infusionsbeutel enthaltend 100 ml 0,9% Kochsalzlösung. Diese Zubereitung zeigt Stabilität über die notwenige Zeitspanne zwischen Präparation und Infusion (ca. 0.3% Hydrolyse nach 4 Stunden bei 25°C). Zum Vergleich: Der reine Wirkstoff in wässriger Lösung bei Raumtemperatur zeigt in vergleichbarer Zeitspanne eine ca. 45%ige Degradation.

### Beispiel 14

Herstellung einer Lösung zur Infusion, enthaltend 430 mg Wirkstoff in 100 ml Infusionslösung:

Zugabe von 8,6 ml oben angegebener Wirkstoff Produkt-Lösung A in einen Infusionsbeutel enthaltend 250 ml 0,9% Kochsalzlösung. Diese Zubereitung zeigt Stabilität über die notwenige Zeitspanne zwischen Präparation und Infusion. Zum Vergleich: Der reine Wirkstoff in wässriger Lösung bei Raumtemperatur zeigt in vergleichbarer Zeitspanne eine ca. 45%ige Degradation.

## Patentansprüche

1. Wässrige pharmazeutische Zubereitung enthaltend 1-(2-Dimethylaminoethyl)-3-((2R,4aS,5R,10bS)-5-phenyl-9-trifluoromethyl-3,4,4a,5,6,10b-hexahydro-2H-pyrano[3,2-c]chinolin-2-ylmethyl)-harnstoff als Wirkstoff, mindestens ein nichtionisches Tensid und mindestens ein mindestens eine Hydroxyl-Gruppe enthaltendes organisches Lösungsmittel.

2. Wässrige pharmazeutische Zubereitung gemäß Anspruch 1, **dadurch gekennzeichnet, dass** das/die nichtionische/s Tensid/e einen HLB-Wert > 8 aufweisen.

3. Wässrige pharmazeutische Zubereitung gemäß Anspruch 2, **dadurch gekennzeichnet, dass** als nichtionische/s Tensid/e Polyoxyethylen-Sorbitan-Fettsäureester, Polyoxyethylen-Polyoxypropylen-Copolymer/e, Polyethylenglycol-Fettsäurester oder/und Polyethylenglykol-Hydroxyfettsäureester enthalten ist/sind.

4. Wässrige pharmazeutische Zubereitung gemäß Anspruch 3, **dadurch gekennzeichnet, dass** als nichtionische/s Tensid/e, Glycerin-Polyethylenglykol-ricinoleat, Polyoxyethylen(20)-sorbitanmonooleat, Polyoxyethylen(20)-sorbitanmonostearat, Polyoxyethylen-Polyoxypropylen-Blockcopolymer und/oder ein Polyethylenglykol(15)hydroxystearat enthalten ist/sind.

5. Wässrige pharmazeutische Zubereitung gemäß einem oder mehreren der Ansprüche 1 bis 4, **dadurch gekennzeichnet, dass** als mindestens eine Hydroxyl-Gruppe enthaltende/s organische/s Lösungsmittel ein- oder mehrwertige/r Alkohol/e und/oder Polyethylenglykol/e enthalten ist/sind.

6. Wässrige pharmazeutische Zubereitung gemäß Anspruch 5, **dadurch gekennzeichnet, dass** als mindestens eine Hydroxyl-Gruppe enthaltende/s organische/s Lösungsmittel, Ethanol, Propylenglykol, Glycerin, Glykofurol und/oder Polyethylenglykol/e enthalten ist/sind.

7. Wässrige pharmazeutische Zubereitung gemäß Anspruch 6, **dadurch gekennzeichnet, dass** als organisches Lösungsmittel Ethanol enthalten ist.

8. Wässrige pharmazeutische Zubereitung gemäß einem oder mehreren der Ansprüche 1 bis 7, **dadurch gekennzeichnet, dass** diese 0,1 bis 20 Gew.-% nichtionische/s Tensid/e und 0,05 bis 20 Gew.-% mindestens ein mindestens eine Hydroxyl-Gruppe enthaltendes organisches Lösungsmittel enthält.

9. Wässrige pharmazeutische Zubereitung gemäß Anspruch 8, **dadurch gekennzeichnet, dass** diese 0,2 bis 5 Gew.-% nichtionische/s Tensid/e und 0,1 bis 5 Gew.-% mindestens ein mindestens eine Hydroxyl-Gruppe enthaltendes organisches Lösungsmittel enthält.

10. Wässrige pharmazeutische Zubereitung gemäß Anspruch 9, **dadurch gekennzeichnet, dass** diese 0,5 bis 2 Gew.-% nichtionische/s Tensid/e und 0,2 bis 2 Gew.-% mindestens ein mindestens eine Hydroxyl-Gruppe enthaltendes organisches Lösungsmittel enthält.

11. Wässrige pharmazeutische Zubereitung gemäß einem oder mehreren der Ansprüche 1 bis 10, **dadurch gekennzeichnet, dass** als nichtionisches Tensid Polyethylenglykol(15)hydroxystearat und als mindestens eine Hydroxyl-Gruppe enthaltendes organisches Lösungsmittel Ethanol enthalten ist.

12. Verfahren zur Herstellung einer wässrigen pharmazeutischen Zubereitung gemäß einem oder mehreren der Ansprüche 1 bis 11, **dadurch gekennzeichnet, dass** eine flüssige pharmazeutische Zubereitung enhaltend mindestens ein mindestens eine Hydroxyl-Gruppe enthaltendes organisches Lösungsmittel und/oder mindestens ein nichtionisches Tensid mit der/den jeweils fehlenden Flüssigkeit/en, d. h. mit Wasser, mit Wasser und mindestens einem nichtionischen Tensid oder mit Wasser und mindestens einem mindestens eine Hydroxyl-Gruppe enthaltenden organischen Lösungsmittel vereinigt wird/werden.

13. Verfahren gemäß Anspruch 12, **dadurch gekennzeichnet, dass** die flüssige pharmazeutische Zubereitung mindestens ein mindestens eine Hydroxyl-Gruppe enthaltendes organisches Lösungsmittel und mindestens ein nichtionisches Tensid enthält.

14. Verfahren gemäß Anspruch 12, **dadurch gekennzeichnet, dass** die flüssige pharmazeutische Zubereitung 95 bis 5 Gew.-% mindestens eine Hydroxyl-Gruppe enthaltende/s organische/s Lösungsmittel und 5 bis 95 Gew.-% nichtionische/s Tensid/e enthält.

15. Verfahren gemäß Anspruch 12, **dadurch gekennzeichnet, dass** die flüssige pharmazeutische Zubereitung 70 bis 30 Gew.-% mindestens eine Hydroxyl-Gruppe enthaltende/s organische/s Lösungsmittel und 30 bis 70 Gew.-% nichtionische/s Tensid/e enthält.

16. Verfahren gemäß Anspruch 12, **dadurch gekennzeichnet, dass** die flüssige pharmazeutische Zubereitung 45 bis 55 Gew.-% mindestens eine Hydroxyl-Gruppe enthaltende/s organische/s Lösungsmittel und 45 bis 55 Gew.-% nichtionische/s Tensid/e enthält.

17. Verfahren nach Anspruch 12, **dadurch gekennzeichnet, dass** die flüssige pharmazeutische Zubereitung, wenn sie als flüssigen Bestandteil nur mindestens ein mindestens eine Hydroxyl-Gruppe enthaltendes organisches Lösungsmittel oder nur mindestens ein nichtionisches Tensid enthält, jeweils mit einer Mischung der fehlenden flüssigen Bestandteile, d. h. mit einer Mischung aus Wasser und mindestens einem nichtionischen Tensid bzw. mit einer Mischung aus Wasser und mindestens einem mindestens eine Hydroxyl-Gruppe enthaltenden organischen Lösungsmittel vereinigt wird.

18. Kit enthaltend ein Behältnis mit einer flüssigen pharmazeutischen Zubereitung enhaltend mindestens ein mindestens eine Hydroxyl-Gruppe enthaltendes organisches Lösungsmittel und/oder mindestens ein nichtionisches Tensid und ein Behältnis mit Wasser geeignet zur Herstellung einer wässrigen pharmazeutischen Zubereitung gemäß einem oder mehreren der Ansprüche 1 bis 11.

## Claims

1. Aqueous pharmaceutical composition comprising 1-(2-dimethylaminoethyl)-3-((2R,4aS,5R,10bS)-5-phenyl-9-trifluoromethyl-3,4,4a,5,6,10b-hexahydro-2H-pyrano[3,2-c]quinolin-2-ylmethyl)urea as active compound, at least one nonionic surfactant and at least one organic solvent containing at least one hydroxyl group.

2. Aqueous pharmaceutical composition according to Claim 1, **characterised in that** the nonionic surfactant(s) has (have) an HLB value > 8.

3. Aqueous pharmaceutical composition according to Claim 2, **characterised in that** the nonionic surfactant(s) present is (are) polyoxyethylene sorbitan fatty acid ester(s), polyoxyethylene-polyoxypropylene copolymer(s), polyethylene glycol fatty acid ester(s) and/or polyethylene glycol hydroxyfatty acid ester(s).

4. Aqueous pharmaceutical composition according to Claim 3, **characterised in that** the nonionic surfactant(s) present is (are) glycerol polyethylene glycol ricinoleate, polyoxyethylene (20) sorbitan monooleate, polyoxyethylene (20) sorbitan monostearate, polyoxyethylene-polyoxypropylene block copolymer and/or polyethylene glycol (15) hydroxystearate.

5. Aqueous pharmaceutical composition according to one or more of Claims 1 to 4, **characterised in that** the organic solvent(s) containing at least one hydroxyl group present is (are) mono- or polyhydric alcohol(s) and/or polyethylene glycol(s).

6. Aqueous pharmaceutical composition according to Claim 5, **characterised in that** the organic solvent(s) containing at least one hydroxyl group present is (are) ethanol, propylene glycol, glycerol, glycofurol and/or polyethylene glycol(s).

7. Aqueous pharmaceutical composition according to Claim 6, **characterised in that** the organic solvent present is ethanol.

8. Aqueous pharmaceutical composition according to one or more of Claims 1 to 7, **characterised in that** it comprises 0.1 to 20% by weight of nonionic surfactant(s) and 0.05 to 20% by weight of at least one organic solvent containing at least one hydroxyl group.

9. Aqueous pharmaceutical composition according to Claim 8, **characterised in that** it comprises 0.2 to 5% by weight of nonionic surfactant(s) and 0.1 to 5% by weight of at least one organic solvent containing at least one hydroxyl group.

10. Aqueous pharmaceutical composition according to Claim 9, **characterised in that** it comprises 0.5 to 2% by weight of nonionic surfactant(s) and 0.2 to 2% by weight of at least one organic solvent containing at least one hydroxyl group.

11. Aqueous pharmaceutical composition according to one or more of Claims 1 to 10, **characterised in that** the nonionic surfactant present is polyethylene glycol (15) hydroxystearate and the organic solvent containing at least one hydroxyl group present is ethanol.

12. Process for the preparation of an aqueous pharmaceutical composition according to one or more of Claims 1 to 11, **characterised in that** a liquid pharmaceutical composition comprising at least one organic solvent containing at least one hydroxyl group and/or at least one nonionic surfactant is combined with the respective missing liquid(s), i.e. with water, with water and at least one nonionic surfactant or with water and at least one organic solvent containing at least one hydroxyl group.

13. Process according to Claim 12, **characterised in that** the liquid pharmaceutical composition comprises at least one organic solvent containing at least one hydroxyl group and at least one nonionic surfactant.

14. Process according to Claim 12, **characterised in that** the liquid pharmaceutical composition comprises 95 to 5% by weight of organic solvent(s) containing at least one hydroxyl group and 5 to 95% by weight of nonionic surfactant(s).

15. Process according to Claim 12, **characterised in that** the liquid pharmaceutical composition comprises 70 to 30% by weight of organic solvent(s) containing at least one hydroxyl group and 30 to 70% by weight of nonionic surfactant(s).

16. Process according to Claim 12, **characterised in that** the liquid pharmaceutical composition comprises 45 to 55% by weight of organic solvent(s) containing at least one hydroxyl group and 45 to 55% by weight of nonionic surfactant(s).

17. Process according to Claim 12, **characterised in that** the liquid pharmaceutical composition, if it comprises only at least one organic solvent containing at least one hydroxyl group or only at least one nonionic surfactant as liquid constituent, is in each case combined with a mixture of the missing liquid constituents, i.e. with a mixture of water and at least one nonionic surfactant or with a mixture of water and at least one organic solvent containing at least one hydroxyl group.

18. Kit comprising a container containing a liquid pharmaceutical composition comprising at least one organic solvent containing at least one hydroxyl group and/or at least one nonionic surfactant and a container containing water which is suitable for the preparation of an aqueous pharmaceutical composition according to one or more of Claims 1 to 11.

## Revendications

1. Composition pharmaceutique aqueuse comprenant de la 1-(2-diméthyl-aminoéthyl)-3-((2R,4aS,5R,10bS)-5-phényl-9-trifluorométhyl-3,4,4a,5,6,10b-hexahydro-2H-pyrano[3,2-c]quinoléin-2-ylméthyl)urée comme composé actif, au moins un agent tensioactif non ionique et au moins un solvant organique contenant au moins un groupement hydroxyle.

2. Composition pharmaceutique aqueuse selon la revendication 1, **caractérisée en ce que** le ou les agents tensioactifs non ioniques possèdent un rapport hydro-lipophile > 8.

3. Composition pharmaceutique aqueuse selon la revendication 2, **caractérisée en ce que** le ou les agents tensioactifs non ioniques présents sont un ou des esters d'acide gras et de sorbitan polyoxyéthylénés, un ou des copolymères de polyoxyéthylène-polyoxypropylène, un ou des esters d'acide gras et de glycol polyéthylénés et/ou un ou des esters d'acide gras hydroxylé et de polyéthylène glycol.

4. Composition pharmaceutique aqueuse selon la revendication 3, **caractérisée en ce que** le ou les agents tensioactifs non ioniques présents sont le ricinoléate de glycérol et de polyéthylène glycol, le monooléate de sorbitan polyoxyéthyléné (20), le monostéarate de sorbitan polyoxyéthyléné (20), le copolymère bloc de polyoxyéthylène-polyoxypropylène et/ou l'hydroxystéarate de polyéthylène glycol (15).

5. Composition pharmaceutique aqueuse selon l'une ou plusieurs parmi les revendications 1 à 4, **caractérisée en ce que** le ou les solvants organiques contenant au moins un groupement hydroxyle présents sont un ou des alcools mono- ou polyhydriques et/ou un ou des polyéthylène glycols.

6. Composition pharmaceutique aqueuse selon la revendication 5, **caractérisée en ce que** le ou les solvants organiques contenant au moins un groupement hydroxyle présents sont l'éthanol, le propylène glycol, le glycérol, le glycofurol et/ou un ou des polyéthylène glycols.

7. Composition pharmaceutique aqueuse selon la revendication 6, **caractérisée en ce que** le solvant organique présent est l'éthanol.

8. Composition pharmaceutique aqueuse selon l'une ou plusieurs parmi les revendications 1 à 7, **caractérisée en ce qu'**elle comprend de 0,1 à 20% en poids d'agent(s) tensioactif(s) non ionique(s) et de 0,05 à 20% en poids d'au moins un solvant organique contenant au moins un groupement hydroxyle.

9. Composition pharmaceutique aqueuse selon la revendication 8, **caractérisée en ce qu'**elle comprend de 0,2 à 5% en poids d'agent(s) tensioactif(s) non ionique(s) et de 0,1 à 5% en poids d'au moins un solvant organique contenant au moins un groupement hydroxyle.

10. Composition pharmaceutique aqueuse selon la revendication 9, **caractérisée en ce qu'**elle comprend de 0,5 à 2% en poids d'agent(s) tensioactif(s) non ionique(s) et de 0,2 à 2% en poids d'au moins un solvant organique contenant au moins un groupement hydroxyle.

11. Composition pharmaceutique aqueuse selon l'une ou plusieurs parmi les revendications 1 à 10, **caractérisée en ce que** l'agent tensioactif non ionique présent est l'hydroxystéarate de polyéthylène glycol (15) et le solvant organique contenant au moins un groupement hydroxyle présent est l'éthanol.

12. Procédé de préparation d'une composition pharmaceutique aqueuse selon l'une ou plusieurs parmi les revendications 1 à 11, **caractérisé en ce qu'**une composition pharmaceutique liquide comprenant au moins un solvant organique contenant au moins un groupement hydroxyle et/ou au moins un agent tensioactif non ionique est combinée avec le ou les liquides manquants respectifs, c'est-à-dire avec de l'eau, avec de l'eau et au moins un agent tensioactif non ionique ou avec de l'eau et au moins un solvant organique contenant au moins un groupement hydroxyle.

13. Procédé selon la revendication 12, **caractérisé en ce que** la composition pharmaceutique liquide comprend au moins un solvant organique contenant au moins un groupement hydroxyle et au moins un agent tensioactif non ionique.

14. Procédé selon la revendication 12, **caractérisé en ce que** la composition pharmaceutique liquide comprend de 95 à 5% en poids de solvant(s) organique(s) contenant au moins un groupement hydroxyle et de 5 à 95% en poids d'agent(s) tensioactif(s) non ionique(s).

15. Procédé selon la revendication 12, **caractérisé en ce que** la composition pharmaceutique liquide comprend de 70 à 30% en poids de solvant(s) organique(s) contenant au moins un groupement hydroxyle et de 30 à 70% en poids d'agent(s) tensioactif(s) non ionique(s).

16. Procédé selon la revendication 12, **caractérisé en ce que** la composition pharmaceutique liquide comprend de 45 à 55% en poids de solvant(s) organique(s) contenant au moins un groupement hydroxyle et de 45 à 55% en poids d'agent(s) tensioactif(s) non ionique(s).

17. Procédé selon la revendication 12, **caractérisé en ce que** la composition pharmaceutique liquide, si elle comprend seulement au moins un solvant organique contenant au moins un groupement hydroxyle ou seulement au moins un agent tensioactif non ionique comme constituant liquide, est dans chaque cas combinée avec un mélange des constituants liquides manquants, c'est-à-dire avec un mélange d'eau et d'au moins un agent tensioactif non ionique ou avec un mélange d'eau et d'au moins un solvant organique contenant au moins un groupement hydroxyle.

18. Kit comprenant un conteneur contenant une composition pharmaceutique liquide comprenant au moins un solvant organique contenant au moins un groupement hydroxyle et/ou au moins un agent tensioactif non ionique et un conteneur contenant de l'eau qui est convenable pour la préparation d'une composition pharmaceutique aqueuse selon l'une ou plusieurs parmi les revendications 1 à 11.
